(19) **Europäisches Patentamt / European Patent Office / Office européen des brevets**

(11) **EP 4 650 443 A1**

(12) **EUROPEAN PATENT APPLICATION**

(43) Date of publication:
**19.11.2025 Bulletin 2025/47**

(21) Application number: **24175431.6**

(22) Date of filing: **13.05.2024**

(51) International Patent Classification (IPC):
**C12N 9/80** *(2006.01)* **A23J 3/34** *(2006.01)*

(52) Cooperative Patent Classification (CPC):
**C12Y 305/01044; A23J 3/34; C12N 9/80**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Novozymes A/S**
**2880 Bagsvaerd (DK)**

(72) Inventor: **XING, Xuelian**
**Tianjin, 300457 (CN)**

(74) Representative: **NVS EPO Representatives**
**Krogshoejvej 36**
**2880 Bagsvaerd (DK)**

Remarks:
The complete document including Reference Table(s) and the Sequence Listing(s) can be downloaded from the EPO website

(54) **STABILIZED LIQUID DEAMIDASE COMPOSITIONS**

(57) The invention provides liquid deamidase compositions comprising mono- or disaccharide(s), which exhibit high enzymatic stability after storage.

**Description**

**Reference to a Sequence Listing**

This application contains a Sequence Listing in computer readable form, which is incorporated herein by reference.

## FIELD OF THE INVENTION

The present invention relates to liquid deamidase compositions comprising mono- or disaccharides, which exhibit high enzymatic stability after storage.

## BACKGROUND

Liquid enzyme compositions are desirous product formats because they are easy to handle in an industrial environment. They can be pumped directly into an industrial process, and generally do not require safety measures to avoid exposure to enzyme dust, as compared to solid enzyme compositions.

While solid enzyme compositions generally exhibit excellent enzymatic storage stability, liquid enzyme compositions are much more challenging, because solubilized enzymes are more fragile than enzymes on solid form.

WO 2013/064736 discloses liquid deamidase compositions comprising glycerol/sorbitol at a pH of 4.4-5.1.

WO 2022/118914 discloses liquid deamidase compositions comprising sorbitol at a pH of 5.5 or more.

## SUMMARY OF THE INVENTION

The present invention provides, in a first aspect, a liquid enzyme composition, comprising

(a) a protein-glutamine glutaminase, and
(b) 10-80% w/w of mono- or disaccharide(s).

Other aspects and embodiments of the invention are apparent from the description and examples.

Unless otherwise indicated, or if it is apparent from the context that something else is meant, all percentages are percentage by weight (% w/w).

As used herein, the term "consists essentially of" (and grammatical variants thereof), as applied to the compositions and methods of the invention, means that the compositions/methods may contain additional components so long as the additional components do not materially alter the composition/method.

As used herein, the term "essentially free of' (and grammatical variants thereof), as applied to the compositions and methods of the invention, means that the compositions/methods may contain minor amounts of the specified component so long as the amount of the component does not materially alter, or provide any material effect on, the composition/method. In an embodiment, "essentially free of" means 0% w/w.

**Sequences**

SEQ ID NO: 1: Amino acid sequence of a deamidase from *Chryseobacterium* sp-62563.

SEQ ID NO: 2: Amino acid sequence of a deamidase propeptide from *Chryseobacterium* sp-62563.

## DETAILED DESCRIPTION

In the context of the invention, the term "deamidase" means a protein-glutamine glutaminase (also known as glutaminylpeptide glutaminase, or protein deamidase) activity, as described in EC 3.5.1.44, which catalyzes the hydrolysis of the gamma-amide of glutamine substituted at the carboxyl position or both the alpha-amino and carboxyl positions, e.g., L-glutaminylglycine and L-phenylalanyl-L-glutaminylglycine. Thus, deamidases can deamidate glutamine residues in proteins to glutamate residues and are also referred to as protein glutamine deamidase. Deamidases comprise a Cys-His-Asp catalytic triad (*e.g.*, Cys-156, His-197, and Asp-217, as shown in Hashizume et al. "Crystal structures of protein glutaminase and its pro forms converted into enzyme-substrate complex", Journal of Biological Chemistry, vol. 286, no. 44, pp. 38691-38702) and belong to the InterPro entry IPR041325. In a preferred embodiment, the deamidases of the present invention belong to PFAM domain PF18626.

Deamidases are catalytic proteins (enzymes), and the term "active (deamidase) enzyme protein" is defined herein as the amount of catalytic protein(s), which exhibits deamidase activity. This can be determined using an activity based analytical enzyme assay. This technique is well-known in the art.

Deamidase activity was measured using the assay described in Example 1. The activity assay consists of two separate de-coupled parts: (1) an enzymatic step wherein ammonia is formed by the catalytic action of the protein deamidase; and (2) a non-enzymatic detection step, wherein the ammonia formed in step (1) is derivatized to a blue indophenol compound with an absorption maximum at 630 nm. The amount of enzyme producing 1 μmol ammonia per minute at 37°C is defined as 1 unit (given in Indophenol Assay Unit: IPA(U)). The activity may be determined relative to a standard of declared strength.

For purposes of the present invention, the sequence identity between two amino acid sequences is determined as the output of "longest identity" using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. In order for the Needle program to report the longest identity, the -nobrief option must be specified in the command line. The output of Needle labeled "longest identity" is calculated as follows:

$$\text{(Identical Residues} \times 100)/(\text{Length of Alignment} - \text{Total Number of Gaps in Alignment})$$

**Liquid enzyme composition**

We have found that aqueous solutions of deamidase enzyme exhibit improved enzyme stability after storage when the solution comprises mono- or disaccharide(s). It is known from the prior art that glycerol and/or sorbitol are useful stabilizers in liquid deamidase compositions. However, we have found that deamidase compositions comprising mono- or disaccharide(s) exhibit even better storage stability. Thus, the present invention provides, in a first aspect, a liquid enzyme composition, comprising

    (a) a protein-glutamine glutaminase, and
    (b) 10-80% w/w of mono- or disaccharide(s).

The mono- or disaccharide(s) may be selected from the group consisting of glucose, fructose, galactose, sucrose, lactose, maltose, trehalose, cellobiose, and combinations thereof.

Preferably, the mono- or disaccharide is a disaccharide, such as sucrose, lactose, maltose, trehalose, and/or cellobiose; in particular sucrose.

In an embodiment, the liquid enzyme composition comprises 10-70% w/w, preferably 10-60% w/w, 20-70% w/w, or 20-60% w/w, of the mono- or disaccharide.

In an embodiment, the liquid enzyme composition has a pH of at least pH 3.5, preferably at least pH 4.0. Preferably the pH is in the range of pH 3.5-10, more preferably in the range of pH 4-9 or in the range of pH 4-8. In an embodiment, the pH of the composition is at most pH 8.

The composition may further comprise at least 10% w/w of water.

In an embodiment, the composition comprises the deamidase (protein-glutamine glutaminase) in an amount of 20-10000 IPA(U) per gram; preferably in an amount of 40-8000 IPA(U) per gram, 60-6000 IPA(U) per gram, 80-5000 IPA(U) per gram, or in an amount of 100-4000 IPA(U) per gram. The amount of deamidase may also be expressed in "active (deamidase) enzyme protein"; thus, the liquid enzyme composition may comprise the deamidase (protein-glutamine glutaminase) in an amount of 0.01-15% w/w of active deamidase enzyme protein; preferably in an amount of 0.05-10% w/w, in an amount of 0.1-5% w/w, or in an amount of 0.1-3% w/w of active deamidase enzyme protein.

As described below, the liquid enzyme composition may further comprise at least 2% w/w, preferably at least 4% w/w, at least 6% w/w, or at least 8% w/w of salt(s).

The liquid enzyme composition may further comprise a reducing agent to avoid oxidation of the cysteine residue in the active site and maintain the deamidase enzymatic activity during storage. The reducing agent may, for example, be a salt of sulfite, metabisulfite, thiosulphate, or ascorbate. The liquid enzyme composition may comprise at least 0.1% w/w of the reducing agent; such as 0.1-5% w/w or 0.1-2% w/w of the reducing agent.

**Mono- or disaccharides**

The liquid enzyme composition comprises 10-80% w/w of mono- or disaccharide(s), such as 10-75% w/w, 10-70% w/w, 10-65% w/w, or 10-60% w/w of mono- or disaccharide(s). In an embodiment, the liquid composition comprises at least 20% w/w, preferably at least 25% w/w, at least 30% w/w, or at least 35% w/w, of mono- or disaccharide(s).

The composition of the invention may further comprise polyol(s) selected from the group consisting of glycerol, ethylene glycol, diethylene glycol, triethylene glycol, propylene glycol, dipropylene glycol, tripropylene glycol, polyethylene glycol (PEG), and sugar alcohols. The polyethylene glycol may have an average molecular weight at or below about 500. Examples of sugar alcohols are sorbitol, mannitol, erythritol, dulcitol, inositol, xylitol, adonitol, isomalt, and maltitol. The composition may comprise such polyols in an amount of less than 40% w/w, less than 30% w/w, less than 20% w/w, less than 10% w/w, or less than 5% w/w.

**Salts**

The liquid enzyme composition may further comprise at least 2% w/w, preferably at least 4% w/w, at least 6% w/w, or at least 8% w/w of salt(s). The amount of salt(s) is calculated as unhydrated salt, thus excluding any complexed water (crystal water). The skilled person will recognize that the upper limit of a salt in the liquid enzyme composition is determined by the solubility of the salt. In an embodiment, the liquid enzyme composition may comprise at most 20% w/w of salt, preferably at most 15% w/w of salt or at most 10% w/w of salt.

The salt(s) may be selected from the group consisting of alkali metal sulfates, carbonates, nitrates, phosphates, halides, formates, acetates, and citrates; alkaline earth metal sulfates, carbonates, nitrates, phosphates, halides, formates, acetates, and citrates; transition metal sulfates, carbonates, nitrates, phosphates, halides, formates, acetates, and citrates; and ammonium sulfates, carbonates, nitrates, halides, formates, acetates, and citrates.

Examples of such salts include sodium sulfate, potassium sulfate, ammonium sulfate, magnesium sulfate, zinc sulfate, sodium carbonate, potassium carbonate, ammonium carbonate, sodium bicarbonate, potassium bicarbonate, ammonium bicarbonate, sodium nitrate, potassium nitrate, ammonium nitrate, magnesium nitrate, zinc nitrate, calcium nitrate, sodium phosphate, potassium phosphate, ammonium phosphate, magnesium phosphate, zinc phosphate, calcium phosphate, sodium chloride, potassium chloride, ammonium chloride, magnesium chloride, zinc chloride, calcium chloride, sodium formate, potassium formate, ammonium formate, magnesium formate, zinc formate, calcium formate, sodium acetate, potassium acetate, ammonium acetate, magnesium acetate, zinc acetate, calcium acetate, sodium citrate, potassium citrate, ammonium citrate, and magnesium citrate. Included are also the hydrates thereof.

Preferred salts are sodium, potassium, ammonium, magnesium, zinc, and calcium salts of formate, acetate, citrate, and chloride. More preferred salts are sodium and potassium salts of formate, acetate, citrate, and chloride.

**Deamidase enzymes**

The deamidase comprised in the liquid enzyme composition of the invention is usually produced by a microbial fermentation and subsequent recovery process. The subsequent recovery process may comprise a maturation/activation step, where an inhibitory propeptide is separated from a deamidase pro-form to produce an active (or more active) deamidase. Thus, the liquid enzyme composition of the invention may further comprise a deamidase inhibitory propeptide, which is not covalently linked to the deamidase. Deamidase inhibitory propeptides are, for example, described in PCT/EP2023/055936. An exemplary propeptide is shown in SEQ ID NO: 2, and others can be identified using protein structure prediction tools (see, for example, Jumper et al., 2021, "Highly accurate protein structure prediction with AlphaFold", Nature 596: 583-589). Such propeptides may be cleaved/separated from the deamidase by the (recombinant) microbial expression organism, or extracellularly by using a suitable site-specific protease. Preferred expression organisms are *Chryseobacterium* and *Bacillus* species.

The fermentation liquid/broth may be subjected to a flocculation/precipitation step to provide a purified deamidase supernatant, and subsequently the purified deamidase supernatant may be subjected to a membrane filtration to provide a concentrated deamidase solution. Preferably, the membrane filtration comprises an ultra-filtration. The concentrated deamidase solution may subsequently be used to produce the liquid composition of the invention in a process that comprises mixing the concentrated deamidase solution with a mono- or disaccharide, and optionally evaporating some water. Water may also be evaporated from the concentrated deamidase solution before adding the mono- or disaccharide(s).

Depending on the desired product concentration, the fermentation broth (from the fermentation), the deamidase supernatant (from the flocculation), or the concentrated deamidase solution (from the membrane filtration) may be subjected to spray-drying (or freeze drying) to provide a deamidase powder. The deamidase powder may subsequently be used to produce the liquid composition of the invention in a process that comprises mixing the deamidase powder with water and polyol.

The deamidase comprised in the liquid enzyme composition of the invention may have an amino acid sequence identity of at least 60%, preferably at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or even 100%, as compared to the amino acid sequence of SEQ ID NO: 1.

Alternatively, the deamidase may have up to 30 alterations (e.g., substitutions, deletions and/or insertions), preferably up to 25, up to 20, up to 15, up to 10, up to 9, up to 8, up to 7, up to 6, up to 5, up to 4, up to 3, up to 2, or up to 1 alteration(s), in particular substitutions, as compared to the amino acid sequence of SEQ ID NO: 1.

Amino acid alterations of both the deamidase and propeptide, as described above, may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding module.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant molecules are tested for enzyme activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide, and/or be inferred from sequence homology and conserved catalytic machinery with a related polypeptide or within a polypeptide or protein family with polypeptides/proteins descending from a common ancestor, typically having similar three-dimensional structures, functions, and significant sequence similarity.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, CRISPR gene editing, phage display (e.g., Lowman et al., 1991, Biochemistry 30: 10832-10837; US 5,223,409; WO 92/06204), and region-directed mutagenesis (Derbyshire et al., 1986, Gene 46: 145; Ner et al., 1988, DNA 7: 127).

**Uses**

Protein deamidase can be applied on almost all types of proteins (plant proteins, animal protein, fermented proteins etc) where the enzyme will lower the isoelectric point of the proteins, and will, when the proteins are applied at a pH above the isoelectric point, improve solubility, electrostatic repulsion, improve different types of functionalities like foaming, emulsification, water binding etc., change affinity to flavours and off-flavours, gelling properties, improve thermostability etc. The enzymatically modified proteins can be applied as ingredients in various foods and beverages or the protein deamidade can be applied directly into a food production process like in a yogurt fermentation.

Plant proteins often have a low solubility and low functional properties. Deamidation is known to improve the solubility of plants proteins and partly of consequence hereof improve the functional properties including foaming activity, foaming stability, emulsification activity and emulsification stability. This has been observed on cross of several plant protein substrates including cereals protein like oat, wheat, corn protein and legume proteins like soy and pea protein, coconut protein etc. Negative attributes associated with the partly insoluble proteins like sandiness and grittiness are being mitigated by the enzymatic deamidation.

For example, enzymatically partly deamidated oat protein becomes essentially fully soluble at neutral pH also leading to significantly improved emulsification properties. (Z-I Jiang at al, J Cereal Science (2015): 64: 126-132). One practical implication is the use of protein deamidase in the process for oat milk production, leading to an oat milk with increased protein content, well suited to meet the requirement for the barista segment (WO 2014/123466). Similarly, emulsification and foaming properties are improved when soy protein isolate is enzymatically deamidated (I Suppavorasatit et al J. Agric. Food Chem (2011) 59: 11621-11628). For enzymatically deamidated pea protein isolate improved solubility, homogeneity, dispersibility, and suspendability and reduced beany flavour, grittiness and lumpiness have been observed (L Fang et al J, Agric. Food Chem. (2020) 68: 1691-1697). Even the highly insoluble corn protein (zein) becomes soluble at pH 5 and 7 and with significantly improved emulsification properties (YH Yong et al. J. Agric Food Chem. (2006): 54: 6034-6040).

The improved functional properties provided by enzymatic deamidation makes the protein deamidase well suited for a variety of food applications of plant protein containing food products like milk analogues with increased protein content, reduced graininess & grittiness, improved mouthfeel, and barista properties, Similarly solutions for the yogurt analogue segments with improved mouthfeel, texture and hydrocolloid replacement. Protein deamidase has also been suggested to improve the texture of plant-based meat analogues and plant-based eggs (X Liu et al Foods (2022) 11: 440).

Deamidation of plant proteins also have a positive impact on the flavour of proteins. Plant proteins are associated with various hydrophobic off-flavours like lipid oxidation products, e.g., having a beany off-flavour, or saponins, phenolics, and flavonoids giving a bitter off-flavour. Enzymatic deamidation of plant proteins reduces the hydrophobicity of the proteins, which therefore reduces the affinity for the hydrophobic off-flavours. Protein deamidase can therefore be applied to improve the flavour of plant proteins by inclusion of the enzyme in the process for recovery of protein concentrates or isolates, or by treatment of recovered proteins like protein isolates (X Liu et al Foods (2022) 11: 440). Flavour improvement is, e.g., demonstrated for soy (I Suppavorasatit et al J. Agric. Food Chem (2012) 60: 7817-7823).

Application of protein deamidase in protein recovery process like the pea protein recovery process leads to improved recovery yield of the proteins, like when applied in the recovery process leading to legume protein concentrate and isolates (WO 2021049591).

Protein deamidase also have several applications on dairy proteins and dairy based foods. Deamidation of whey lead to a better electrostatic repulsion of the proteins, giving a better thermostability, avoiding undesirable aggregation in whey protein solutions (e.g., in protein fortified beverages) when the protein solution is heat-treated (N Miwa et al J. Agric. Food Chem (2013) 61: 2205-2212). Enzymatic deamidation in skim milk leads to much improved solubility, viscosity and provides a translucent milk drink (N Miwa et al International dairy journal (2010) 20: 393-399). Application of protein deamidase in the yogurt process leads to an improved stabilization, which can e.g. be applied to replace pectin and other hydrocolloids in drinking yogurt.

Protein (glutaminase) deamidase can be applied together with other enzymes including other enzymes modifying or degrading protein. Combinations between protein glutamine deamidase and protein asparagine deamidase can provide a higher degree of deamidation of the proteins and thereby an even better applicational performance. Protein deamidase can be applied together with protein crosslinking enzymes like transglutaminase, where the crosslinking of the protein is modified, partly as the transglutaminase will be prevented from reacting with the glutamines which have been converted to glutamic acid by the deamidase. When a combination of transglutaminase and protein deamidase is used in the yogurt process, a texturing effect is obtained, applicable to replace added dairy proteins or hydrocolloids, providing a yogurt with a smooth texture and avoiding the lumpy texture which is seen when transglutaminase is used alone. A similar effect is observed when this enzyme combination is used for production of plant-based yogurt analogues. Furthermore, the protein deamidase can be applied together with proteases where the resulting protein hydrolysate will have improved solubility and taste and changed functional properties.

Further embodiments of the invention include:

Embodiment 1. A liquid enzyme composition, comprising

  (a) a protein-glutamine glutaminase, and
  (b) 10-80% w/w of mono- or disaccharide(s).

Embodiment 2. The liquid enzyme composition of the preceding embodiment, which comprises the protein-glutamine glutaminase in an amount of 20-10000 IPA(U) per gram.

Embodiment 3. The liquid enzyme composition of any of the preceding embodiments, which comprises the protein-glutamine glutaminase in an amount of 40-8000 IPA(U) per gram.

Embodiment 4. The liquid enzyme composition of any of the preceding embodiments, which comprises the protein-glutamine glutaminase in an amount of 60-6000 IPA(U) per gram.

Embodiment 5. The liquid enzyme composition of any of the preceding embodiments, which comprises the protein-glutamine glutaminase in an amount of 80-5000 IPA(U) per gram.

Embodiment 6. The liquid enzyme composition of any of the preceding embodiments, which comprises the protein-glutamine glutaminase in an amount of 100-4000 IPA(U) per gram.

Embodiment 7. The liquid enzyme composition of any of the preceding embodiments, which comprises the protein-glutamine glutaminase in an amount of 0.01-15% w/w of active deamidase enzyme protein.

Embodiment 8. The liquid enzyme composition of any of the preceding embodiments, which comprises the protein-glutamine glutaminase in an amount of 0.05-10% w/w of active deamidase enzyme protein.

Embodiment 9. The liquid enzyme composition of any of the preceding embodiments, which comprises the protein-glutamine glutaminase in an amount of 0.05-5% w/w of active deamidase enzyme protein.

Embodiment 10. The liquid enzyme composition of any of the preceding embodiments, which comprises the protein-glutamine glutaminase in an amount of 0.1-5% w/w of active deamidase enzyme protein.

Embodiment 11. The liquid enzyme composition of any of the preceding embodiments, which comprises the protein-glutamine glutaminase in an amount of 0.1-3% w/w of active deamidase enzyme protein.

Embodiment 12. The liquid enzyme composition of any of the preceding embodiments, which comprises the protein-glutamine glutaminase in an amount of 0.1-2% w/w of active deamidase enzyme protein.

Embodiment 13. The liquid enzyme composition of any of the preceding embodiments, where the protein-glutamine glutaminase belongs to EC 3.5.1.44.

Embodiment 14. The liquid enzyme composition of any of the preceding embodiments, where the protein-glutamine glutaminase has an amino acid sequence identity of at least 60%, preferably at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or even 100%, as compared to the amino acid sequence of SEQ ID NO: 1.

Embodiment 15. The liquid enzyme composition of any of the preceding embodiments, where the protein-glutamine glutaminase has up to 30 alterations (e.g., substitutions, deletions and/or insertions), preferably up to 25, up to 20, up to 15, up to 10, up to 9, up to 8, up to 7, up to 6, up to 5, up to 4, up to 3, up to 2, or up to 1 alteration(s), in particular substitutions, as compared to the amino acid sequence of SEQ ID NO: 1.

Embodiment 16. The liquid enzyme composition of any of the preceding embodiments, which comprises the mono- or disaccharide in an amount of 10-75% w/w.

Embodiment 17. The liquid enzyme composition of any of the preceding embodiments, which comprises the mono- or disaccharide in an amount of 10-70% w/w.

Embodiment 18. The liquid enzyme composition of any of the preceding embodiments, which comprises the mono- or disaccharide in an amount of 10-65% w/w.

Embodiment 19. The liquid enzyme composition of any of the preceding embodiments, which comprises the mono- or disaccharide in an amount of 10-60% w/w.

Embodiment 20. The liquid enzyme composition of any of the preceding embodiments, which comprises the mono- or disaccharide in an amount of at least 20% w/w.

Embodiment 21. The liquid enzyme composition of any of the preceding embodiments, which comprises the mono- or disaccharide in an amount of at least 25% w/w.

Embodiment 22. The liquid enzyme composition of any of the preceding embodiments, which comprises the mono- or disaccharide in an amount of at least 30% w/w.

Embodiment 23. The liquid enzyme composition of any of the preceding embodiments, which comprises the mono- or disaccharide in an amount of at least 35% w/w.

Embodiment 24. The liquid enzyme composition of any of the preceding embodiments, where the mono- or disaccharide is selected from the group consisting of glucose, fructose, galactose, sucrose, lactose, maltose, trehalose, cellobiose, and combinations thereof.

Embodiment 25. The liquid enzyme composition of any of the preceding embodiments, where the mono- or disaccharide is a disaccharide.

Embodiment 26. The liquid enzyme composition of any of the preceding embodiments, where the pH of the composition is at least pH 3.5.

Embodiment 27. The liquid enzyme composition of any of the preceding embodiments, where the pH of the composition is at least pH 4.0.

Embodiment 28. The liquid enzyme composition of any of the preceding embodiments, where the pH of the composition is at most pH 8.0.

Embodiment 29. The liquid enzyme composition of any of the preceding embodiments, where the pH of the composition is in the range of pH 3.5-10.

Embodiment 30. The liquid enzyme composition of any of the preceding embodiments, where the pH of the composition is in the range of pH 4-9.

Embodiment 31. The liquid enzyme composition of any of the preceding embodiments, where the pH of the composition is in the range of pH 4-8.

Embodiment 32. The liquid enzyme composition of any of the preceding embodiments, which further comprise polyol(s) selected from the group consisting of glycerol, ethylene glycol, diethylene glycol, triethylene glycol, propylene glycol, dipropylene glycol, tripropylene glycol, polyethylene glycol (PEG), and sugar alcohols.

Embodiment 33. The liquid enzyme composition of the preceding embodiment, which comprise the polyol(s) in an amount of less than 40% w/w, less than 30% w/w, less than 20% w/w, less than 10% w/w, or less than 5% w/w.

Embodiment 34. The liquid enzyme composition of any of the preceding embodiments, which further comprises at least 2% w/w of salt.

Embodiment 35. The liquid enzyme composition of any of the preceding embodiments, which comprises at least 4% w/w of salt.

Embodiment 36. The liquid enzyme composition of any of the preceding embodiments, which further comprises at least 6% w/w of salt.

Embodiment 37. The liquid enzyme composition of any of the preceding embodiments, which further comprises at least 8% w/w of salt.

Embodiment 38. The liquid enzyme composition of any of the preceding embodiments, which further comprises at most 20% w/w of salt.

Embodiment 39. The liquid enzyme composition of any of the preceding embodiments, which further comprises at most 15% w/w of salt.

Embodiment 40. The liquid enzyme composition of any of the preceding embodiments, which further comprises at most 10% w/w of salt.

Embodiment 41. The liquid enzyme composition of any of embodiments 34-40, where the salt is selected from the group consisting of sodium sulfate, potassium sulfate, ammonium sulfate, magnesium sulfate, zinc sulfate, sodium carbonate, potassium carbonate, ammonium carbonate, sodium bicarbonate, potassium bicarbonate, ammonium bicarbonate, sodium nitrate, potassium nitrate, ammonium nitrate, magnesium nitrate, zinc nitrate, calcium nitrate, sodium phosphate, potassium phosphate, ammonium phosphate, magnesium phosphate, zinc phosphate, calcium phosphate, sodium chloride, potassium chloride, ammonium chloride, magnesium chloride, zinc chloride, calcium chloride, sodium formate, potassium formate, ammonium formate, magnesium formate, zinc formate, calcium formate, sodium acetate, potassium acetate, ammonium acetate, magnesium acetate, zinc acetate, calcium acetate, sodium citrate, potassium citrate, ammonium citrate, magnesium citrate, and hydrates thereof.

Embodiment 42. The liquid enzyme composition of any of embodiments 34-40, where the salt is selected from the group consisting of sodium chloride, potassium chloride, ammonium chloride, magnesium chloride, zinc chloride, calcium chloride, sodium formate, potassium formate, ammonium formate, magnesium formate, zinc formate, calcium formate, sodium acetate, potassium acetate, ammonium acetate, magnesium acetate, zinc acetate, calcium acetate, sodium citrate, potassium citrate, ammonium citrate, magnesium citrate, and hydrates thereof.

Embodiment 43. The liquid enzyme composition of any of embodiments 34-40, where the salt is selected from the group consisting of sodium chloride, potassium chloride, sodium formate, potassium formate, sodium acetate, potassium acetate, sodium citrate, potassium citrate, and hydrates thereof.

Embodiment 44. The liquid enzyme composition of any of the preceding embodiments, which further comprises a reducing agent.

Embodiment 45. The liquid enzyme composition of any of the preceding embodiments, which further comprises a reducing agent in an amount of at least 0.1% w/w.

Embodiment 46. The liquid enzyme composition of any of the preceding embodiments, which further comprises a reducing agent in an amount of at least 0.1-5% w/w.

Embodiment 47. The liquid enzyme composition of any of the preceding embodiments, which further comprises a reducing agent in an amount of at least 0.1-2% w/w.

Embodiment 48. The liquid enzyme composition of any of the preceding embodiments, which further comprises a salt of sulfite, metabisulfite, thiosulphate, or ascorbate.

Embodiment 49. The liquid enzyme composition of any of the preceding embodiments, which further comprises a salt of sulfite, metabisulfite, thiosulphate, or ascorbate, in an amount of at least 0.1% w/w.

Embodiment 50. The liquid enzyme composition of any of the preceding embodiments, which further comprises a salt of sulfite, metabisulfite, thiosulphate, or ascorbate, in an amount of at least 0.1-5% w/w.

Embodiment 51. The liquid enzyme composition of any of the preceding embodiments, which further comprises a salt of sulfite, metabisulfite, thiosulphate, or ascorbate, in an amount of at least 0.1-2% w/w.

Embodiment 52. A method for preparing the liquid enzyme composition of any of the preceding embodiments, comprising recovering the protein-glutamine glutaminase from a microbial fermentation broth; and mixing the protein-glutamine glutaminase with the mono- or disaccharide.

Embodiment 53. The method of the preceding embodiment, where the microbial fermentation broth is a bacterial fermentation broth.

Embodiment 54. The method of the preceding embodiment, where the microbial fermentation broth is a bacterial fermentation broth comprising a *Chryseobacterium* or *Bacillus* species.

Embodiment 55. The method of any of the preceding embodiments, where the recovery comprises a membrane filtration, such as an ultra-filtration.

Embodiment 56. A method for deamidating a glutamine residue in a plant or milk protein, comprising contacting the plant or milk protein with the liquid enzyme composition of any of the preceding embodiments.

Embodiment 57. The method of the preceding embodiment, where the plant protein is derived from cereals or legumes, and the milk protein is whey protein; more preferably the plant protein is derived from oat, wheat, corn, soy, pea or almond.

## EXAMPLES

Chemicals were commercial products of at least reagent grade.

The deamidase used in the examples is derived from *Chryseobacterium* sp-62563. The amino acid sequence of the deamidase is shown as SEQ ID NO: 1.

## EXAMPLE 1

**Deamidase activity assay**

The deamidase activity assay consists of two separate de-coupled parts:

1) an enzymatic step wherein ammonia is formed by the catalytic action of the deamidase; and
2) a non-enzymatic detection step wherein the ammonia formed in step (1) is derivatized to a blue indophenol compound with an absorption maximum at 630 nm.

In step (1), the ammonia is developed by the deamidating action of the deamidase. In step (2), the generated ammonia reacts with phenol to form dioxyphenylamine under alkaline conditions. The reaction is catalyzed by sodium pentacyanonitrosylferrate(III) (sodium nitroprusside). "Color Reagent solution A" contains phenol and sodium nitroprusside. "Color Reagent Solution B" provides alkaline reaction conditions. The intermediate is then oxidized by addition of sodium hypochlorite ("Color Reagent Solution C") to form indophenol blue. This compound absorbs visible light at 630 nm. The enzyme activity is then calculated using a standard curve.

Assay Procedure

*Step (1) Enzymatic step with ammonia formation*

Reagents:

Assay dilution solution: 0.2 M Na-phosphate buffer, 0.01% Triton X-100, pH 6.5.
Assay buffer: Same as above. Used to prepare stock solution and diluted sample of protein deamidase (referred to in the following as "enzyme").
Substrate solution: 30 mM Z-Gln-Gly (Merck C6154-1G) in assay dilution solution (check pH after dissolution).
Stop solution: 0.4 M TCA.
Standard: $NH_4Cl$ (Ammonium Standard for IC, Merck 59755-100ML, 1000 mg/L $NH_4^+$ in water) diluted in assay dilution solution (see also "Standard curve" section).

Dissolve/dilute enzyme product in assay buffer and prepare suitable dilution resulting in a linear assay response.
Incubation:

1. Add 10 μL of diluted enzyme samples in triplicates to the wells of a 96-well microtiter plate (MTP).
2. Add 100 μL of substrate solution to each well.
3. For blank samples add 100 μL 0.4 M TCA solution.
4. Seal the plate using transparent plate sealer.
5. Incubate the plate for 10 minutes at 37°C, 500 rpm, on a thermomixer equipped with a lid heating function.
6. To stop the reaction, carefully add 100 μL 0.4 M TCA solution (except for the blank samples, which already contain TCA).

Total reaction volume: 210 μL

*Step (2) Ammonia detection step*

Reagents:

Color reagent A: 4% (w/v) Phenol, 0.015% (w/v) sodium pentacyanonitrosylferrate(III) dihydrate (sodium nitroprusside) ($Na_2[Fe(CN)_5NO]·2H_2O$).
Color reagent B: 5% (w/v) Potassium hydroxide.
Color reagent C: 28% (w/v) Potassium carbonate, 6% (v/v) sodium hypochlorite (Sigma-Aldrich 239305-25ml, < 5% available $Cl_2$).

Incubation:

1. Transfer 15 μL from each well from step (1) into a new 96-well MTP.
2. Transfer 45 μL Milli-Q water to each well.
3. To each well, add 30 μL of color reagent B (on lab table, shake gently by hand to mix).
4. To each well, add 60 μL of color reagent A (on lab table, shake gently by hand to mix).

5. To each well, add 60 μL of color reagent C (on lab table, shake gently by hand to mix).
6. Color development: Carefully seal the plate and leave it on lab table for 30 minutes.
7. Carefully transfer the MTP to a plate reader and measure absorbance at 630 nm.

Total reaction volume: 210 μL

Standard curve:

Standard stock solution: 1000 mg $NH_4^+$/L.

The standard curve is prepared by adding dilutions of the ammonium standard in the assay dilution buffer in the ammonia detection step. That is, mixing 15 μL diluted ammonia standard with 45 μL water and then add the color reagents in the order given above; B, A, and C.

The amount of enzyme producing 1 μmol ammonia per minute at 37°C is defined as 1 unit (Indophenol Assay Unit; IPA(U)):

$$\frac{IPA(U)}{mL} = C_{NH4^+}\left(\frac{mg}{L}\right) * \frac{1\ mol}{18.04\ g} * \frac{V_{reaction}\ (\mu L)}{V_{enzyme}\ (\mu L)} * \frac{1}{10\ min.} * \frac{V_{NH3\ detection}\ (\mu L)}{V_{NH3\ generated}\ (\mu L)} * DF$$

where

$$C_{NH4^+}\left(\frac{mg}{L}\right) = C_{NH4^+}^{predil.}\left(\frac{mg}{L}\right) * \frac{V_{NH4^+,predil.}\ (\mu L)}{V_{NH3\ detection}(\mu L)}$$

which can be shortened to

$$\frac{IPA(U)}{mL} = C_{NH4^+}^{predil.}\left(\frac{mg}{L}\right) * \frac{21}{180.04} * DF\ \frac{\mu mol}{mL * min.}$$

where

- $C_{NH4^+}$ is the ammonia concentration in the reaction solution derived from the ammonium standard curve (i.e., taking into account the dilution of the prediluted ammonium standard solution in the ammonia derivatization step).
- 18.04 is the molecular mass of ammonium used for the standard solution.
- $V_{reaction}$ is the reaction volume in the well when ammonia is generated (210 μL).
- $V_{enzyme}$ is the volume of enzyme solution added to the well when ammonia is generated (10 μL).
- $V_{NH3}$ *detection* is the reaction volume in the well when ammonia is detected (210 μL).

**EXAMPLE 2**

**Deamidase storage stability I**

In the first study, the enzymatic stability of several liquid deamidase compositions were evaluated after storage for 4 days at 40°C. All compositions contained the deamidase in an amount of approx. 1080 IPA(U)/g.

The compositions contained 10% w/w NaCl and either 40% w/w glycerol (reference) or 40% w/w sucrose. The pH of the compositions was adjusted to pH 3.8, pH 4.2, or pH 4.5. The activity loss was defined as the lost activity relative to the activity at the time of preparation and incubation of the samples. The lower activity loss, the better enzyme stability.

Table 1. Compositions and activity loss after incubation for 4 days at 40°C.

| pH | Activity loss | |
|---|---|---|
| | Glycerol (reference) | Sucrose |
| 3.8 | 4.4% | 2.4% |
| 4.2 | 3.4% | 2.2% |

(continued)

| pH | Activity loss | |
|---|---|---|
| | Glycerol (reference) | Sucrose |
| 4.5 | 3.0% | 1.5% |

## EXAMPLE 3

**Deamidase storage stability II**

The setup is the same as in Example 1, but the samples were adjusted to pH 4.0 and evaluated after storage for 2 days at 50°C or 60°C.

Table 2. Compositions and activity loss after incubation for 2 days at pH 4.0.

| Temperature | Activity loss | |
|---|---|---|
| | Glycerol (reference) | Sucrose |
| 50°C | 7.3% | 4.9% |
| | 7.8% | 4.7% |
| 60°C | 91.2% | 12.2% |
| | 99.3% | 16.3% |

### Conclusions from Examples 2 and 3

As shown in Tables 1-2, stabilization of the liquid deamidase compositions with sucrose, as compared to glycerol, consistently improves the residual deamidase activity after storage.

## Claims

1.  A liquid enzyme composition, comprising

    (a) a protein-glutamine glutaminase,
    (b) 10-80% w/w of mono- or disaccharide(s).

2.  The liquid enzyme composition of the preceding claim, which comprises the protein-glutamine glutaminase in amount of 20-10000 IPA(U)/g

3.  The liquid enzyme composition of any of the preceding claims, where the protein-glutamine glutaminase belongs to EC 3.5.1.44.

4.  The liquid enzyme composition of any of the preceding claims, which further comprises at least 10% w/w of water.

5.  The liquid enzyme composition of any of the preceding claims, where the pH of the composition is at least pH 3.5, preferably at least pH 4.0, more preferably in the range of pH 3.5-10, even more preferably in the range of pH 4-9 or in the range of pH 4-8.

6.  The liquid enzyme composition of any of the preceding claims, where the mono- or disaccharide is selected from the group consisting of glucose, fructose, galactose, sucrose, lactose, maltose, trehalose, cellobiose, and combinations thereof.

7.  The liquid enzyme composition of any of the preceding claims, where the mono- or disaccharide is a disaccharide; preferably selected from the group consisting of sucrose, lactose, maltose, trehalose, cellobiose, and combinations thereof.

8. The liquid enzyme composition of any of the preceding claims, which comprises 10-70% w/w of the mono- or disaccharide, preferably 20-70% w/w or 20-60% w/w of the mono- or disaccharide.

9. The liquid enzyme composition of any of the preceding claims, which further comprises at least 2% w/w of salt; preferably at least 4% w/w, at least 6% w/w, or at least 8% w/w of salt.

10. The liquid enzyme composition of any of the preceding claims, which comprises at most 20% w/w of salt, preferably at most 15% w/w or at most 10% w/w of salt.

11. The liquid enzyme composition of any of the preceding claims, where the salt is selected from the group consisting of sodium chloride, potassium chloride, ammonium chloride, magnesium chloride, zinc chloride, calcium chloride, sodium formate, potassium formate, ammonium formate, magnesium formate, zinc formate, calcium formate, sodium acetate, potassium acetate, ammonium acetate, magnesium acetate, zinc acetate, calcium acetate, sodium citrate, potassium citrate, ammonium citrate, magnesium citrate, and hydrates thereof.

12. The liquid enzyme composition of any of the preceding claims, which further comprises less than 40% w/w, preferably less than 30% w/w, less than 20% w/w, or less than 10% w/w, of polyol(s) selected from the group consisting of glycerol, ethylene glycol, diethylene glycol, triethylene glycol, propylene glycol, dipropylene glycol, tripropylene glycol, polyethylene glycol (PEG), and sugar alcohols.

13. A method for preparing the liquid enzyme composition of any of the preceding claims, comprising recovering the protein-glutamine glutaminase from a microbial fermentation broth; and mixing the recovered protein-glutamine glutaminase with the mono- or disaccharide.

14. The method of the preceding claim, where the microbial fermentation broth comprises a *Chryseobacterium* or *Bacillus* species; and/or where the recovery comprises a membrane filtration.

15. A method for deamidating a glutamine residue in a plant or milk protein, comprising contacting the plant or milk protein with the liquid enzyme composition of any of the preceding claims; preferably the plant protein is derived from cereals or legumes, and the milk protein is whey protein; more preferably the plant protein is derived from oat, wheat, corn, soy, pea or almond.

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2024/026332 A1 (NORIFUNE SAKI [JP] ET AL) 25 January 2024 (2024-01-25) * Whole document, especially 0038 and claims * | 1-15 | INV. C12N9/80 A23J3/34 |
| A | US 2014/287098 A1 (RAJAKARI KIRSI [FI] ET AL) 25 September 2014 (2014-09-25) * Whole document, especially 0023 and claims * | 1-15 | |
| A | EP 4 289 943 A1 (AMANO ENZYME INC [JP]) 13 December 2023 (2023-12-13) * Whole document, especially 0098, 0101-0103 * | 1-15 | |
| A | LIU XIAO ET AL: "Application Prospect of Protein-Glutaminase in the Development of Plant-Based Protein Foods", FOODS, vol. 11, no. 3, 2 February 2022 (2022-02-02), page 440, XP093028940, DOI: 10.3390/foods11030440 * the whole document * | 1-15 | **TECHNICAL FIELDS SEARCHED (IPC)** C12N A23J |

The present search report has been drawn up for all claims

| Place of search | Date of completion of the search | Examiner |
|---|---|---|
| The Hague | 18 October 2024 | Kools, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 5431

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2024

| Patent document cited in search report | Publication date | Patent family member(s) | | Publication date |
|---|---|---|---|---|
| US 2024026332 A1 | 25-01-2024 | CN | 116547381 A | 04-08-2023 |
| | | EP | 4257682 A1 | 11-10-2023 |
| | | JP | WO2022118914 A1 | 09-06-2022 |
| | | US | 2024026332 A1 | 25-01-2024 |
| | | WO | 2022118914 A1 | 09-06-2022 |
| US 2014287098 A1 | 25-09-2014 | AU | 2012331034 A1 | 26-06-2014 |
| | | BR | 112014010222 A2 | 13-06-2017 |
| | | CA | 2853711 A1 | 10-05-2013 |
| | | CL | 2014001100 A1 | 05-09-2014 |
| | | CN | 104024406 A | 03-09-2014 |
| | | CN | 108676782 A | 19-10-2018 |
| | | DK | 2773753 T3 | 10-12-2018 |
| | | EP | 2773753 A1 | 10-09-2014 |
| | | ES | 2697675 T3 | 25-01-2019 |
| | | FI | 20116074 A | 02-05-2013 |
| | | JP | 6297979 B2 | 20-03-2018 |
| | | JP | 2014532421 A | 08-12-2014 |
| | | KR | 20140096090 A | 04-08-2014 |
| | | MX | 356454 B | 30-05-2018 |
| | | PL | 2773753 T3 | 29-03-2019 |
| | | RU | 2014121318 A | 10-12-2015 |
| | | US | 2014287098 A1 | 25-09-2014 |
| | | WO | 2013064736 A1 | 10-05-2013 |
| | | ZA | 201403965 B | 27-05-2015 |
| EP 4289943 A1 | 13-12-2023 | CN | 116829719 A | 29-09-2023 |
| | | EP | 4289943 A1 | 13-12-2023 |
| | | JP | WO2022168927 A1 | 11-08-2022 |
| | | US | 2024124912 A1 | 18-04-2024 |
| | | WO | 2022168927 A1 | 11-08-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013064736 A
- WO 2022118914 A
- EP 2023055936 W
- WO 9517413 A
- WO 9522625 A
- US 5223409 A
- WO 9206204 A
- WO 2014123466 A
- WO 2021049591 A

### Non-patent literature cited in the description

- **HASHIZUME et al.** Crystal structures of protein glutaminase and its pro forms converted into enzyme-substrate complex. *Journal of Biological Chemistry*, vol. 286 (44), 38691-38702
- **NEEDLEMAN ; WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **JUMPER et al.** Highly accurate protein structure prediction with AlphaFold. *Nature*, 2021, vol. 596, 583-589
- **CUNNINGHAM ; WELLS**. *Science*, 1989, vol. 244, 1081-1085
- **HILTON et al.** *J. Biol. Chem.*, 1996, vol. 271, 4699-4708
- **DE VOS et al.** *Science*, 1992, vol. 255, 306-312
- **SMITH et al.** *J. Mol. Biol.*, 1992, vol. 224, 899-904
- **WLODAVER et al.** *FEBS Lett.*, 1992, vol. 309, 59-64
- **REIDHAAR-OLSON ; SAUER**. *Science*, 1988, vol. 241, 53-57
- **BOWIE ; SAUER**. *Proc. Natl. Acad. Sci. USA*, 1989, vol. 86, 2152-2156
- **LOWMAN et al.** *Biochemistry*, 1991, vol. 30, 10832-10837
- **DERBYSHIRE et al.** *Gene*, 1986, vol. 46, 145
- **NER et al.** *DNA*, 1988, vol. 7, 127
- **Z-I JIANG**. *J Cereal Science*, 2015, vol. 64, 126-132
- **I SUPPAVORASATIT et al.** *J. Agric. Food Chem*, 2011, vol. 59, 11621-11628
- **L FANG et al.** *J, Agric. Food Chem.*, 2020, vol. 68, 1691-1697
- **YH YONG et al.** *J. Agric Food Chem.*, 2006, vol. 54, 6034-6040
- **X LIU et al.** *Foods*, 2022, vol. 11, 440
- **I SUPPAVORASATIT et al.** *J. Agric. Food Chem*, 2012, vol. 60, 7817-7823
- **N MIWA et al.** *J. Agric. Food Chem*, 2013, vol. 61, 2205-2212
- **N MIWA et al.** *International dairy journal*, 2010, vol. 20, 393-399